(19) Europäisches Patentamt
European Patent Office
Office européen des brevets

(11) Veröffentlichungsnummer: **0 218 085**
**A1**

(12) # EUROPÄISCHE PATENTANMELDUNG

(21) Anmeldenummer: **86111971.7**

(22) Anmeldetag: **29.08.86**

(51) Int. Cl.⁴: **C 07 C 143/74**

(30) Priorität: **06.09.85 DE 3531788**

(43) Veröffentlichungstag der Anmeldung: **15.04.87 Patentblatt 87/16**

(84) Benannte Vertragsstaaten: **BE CH DE FR GB IT LI NL**

(71) Anmelder: **HOECHST AKTIENGESELLSCHAFT, Postfach 80 03 20, D-6230 Frankfurt am Main 80 (DE)**

(72) Erfinder: **Günther, Dieter, Dr., Nachtigallenweg 1A, D-6233 Kelkheim (Taunus) (DE)**

(54) **3-Halogen-acetonsulfonamide und Verfahren zu deren Herstellung sowie ihre Verwendung.**

(57) Die Erfindung betrifft neue 3-Halogen-acetonsulfonamide der Formel $X-CH_2-CO-CH_2-SO_2NH_2$ mit X = F, Cl, Br oder J, sowie deren Herstellung und Verwendung. Die Herstellung der erfindungsgemäßen Bromverbindung erfolgt durch Umsetzung von Acetonsulfonamid mit Brom in wäßriger Lösung. Die Fluor-, Chlor- bzw. Jodverbindung wird durch Umsetzung der Bromverbindung mit einem Fluorid, Chlorid bzw. Jodid in einem aprotischen Lösungsmittel hergestellt. Die erfindungsgemäßen Verbindungen können bei der Herstellung von Azofarbstoffen, Pflanzenschutzmitteln, Pharmazeutika und Textilhilfsmitteln verwendet werden.

EP 0 218 085 A1

HOECHST AKTIENGESELLSCHAFT HOE 85/F 180 Dr.MA/sch

# 3-Halogen-acetonsulfonamide und Verfahren zu deren Herstellung sowie ihre Verwendung

Ein Gegenstand der vorliegenden Erfindung sind neue 3-Halogen-acetonsulfonamide der allgemeinen Formel I

$$X-CH_2-\overset{O}{\overset{\|}{C}}-CH_2-SO_2NH_2 \qquad (I)$$

in der X für F, Cl, Br oder J steht. Ein weiterer Gegenstand der Erfindung ist ein Verfahren zur Herstellung der Verbindung I mit X = Br, dadurch gekennzeichnet, daß man Acetonsulfonamid in wäßriger Lösung mit Brom umsetzt. Ein weiterer Gegenstand der Erfindung ist ein Verfahren zur Herstellung von Verbindungen der Formel

$$X-CH_2-\overset{O}{\overset{\|}{C}}-CH_2-SO_2NH_2$$

mit X = F, Cl oder J, dadurch gekennzeichnet, daß man 3-Brom-acetonsulfonamid in einem aprotischen Lösungsmittel mit einem Fluorid, Chlorid oder Jodid umsetzt.

Die erfindungsgemäßen Verbindungen stellen infolge ihrer Reaktivität interessante Zwischenprodukte z.B. zur Herstellung von Azofarbstoffen oder -pigmenten, Pflanzenschutzmitteln, Pharmazeutika oder Textilhilfsmitteln dar.

Man erhält die Bromverbindung der Formel I, wie gesagt, auf sehr einfache Weise, indem man Acetonsulfonamid in wäßriger Lösung bromiert. Überraschenderweise tritt das Brom nicht, wie erwartet, an das aktivierte Kohlenstoffatom in 2-Stellung des Ausgangsprodukts und es entstehen auch keine N-Brom-Derivate.

Die Fluor-, Chlor- bzw. Jodverbindung der Formel I stellt man durch nukleophilen Austausch aus der Bromverbindung in aprotischen dipolaren Medien her.

Die erfindungsgemäße Herstellung von 3-Brom-acetonsulfonamid wird im allgemeinen bei Temperaturen von 0° bis 100°C mit molaren Mengen Brom durchgeführt. Vorzugsweise wählt man jedoch eine Temperatur im Bereich von 20° bis 50°C.

Die Reaktionszeit richtet sich dabei nach der angewendeten Temperatur und ist an der vollständigen Entfärbung des Reaktionsansatzes zu erkennen.

Das als Ausgangsmaterial eingesetzte Acetonsulfonamid kann nach DE-OS 3 323 510 oder US-PS 4 448 988 erhalten werden. Der nukleophile Austausch des Broms im 3-Bromacetonsulfonamid zur Herstellung der drei anderen Halogenacetonsulfonamide der Formel I wird im allgemeinen bei Temperaturen zwischen 20 und 100°C mit Fluoriden, Chloriden bzw. Jodiden in aprotischen Lösungsmitteln, wie Aceton, Dimethylformamid, N-Methyl-pyrrolidon oder Dimethylsulfoxid durchgeführt. Dabei setzt man vorzugsweise Fluoride, Chloride bzw. Jodide von Alkali- oder Erdalkalimetallen oder von Eisen, Aluminium oder Zink ein. Im allgemeinen verwendet man etwa 1 bis 5 Mol Halogenid pro Mol 3-Bromacetonsulfonamid.

Die erfindungsgemäßen Halogenverbindungen sind neue reaktive Substanzen, die als Zwischenprodukte für Pflanzenschutzmittel, Pharmazeutika oder Textilhilfsmittel geeignet und zahlreichen weiteren Umsetzungen zugänglich sind. Zum Beispiel eröffnet ihre Reaktion mit 2-Amino-N-heterocyclen, wie beispielsweise 2-Amino-pyridin, einen Zugang zu mit Sulfonamidgruppen substituierten Bicyclen wie Imidazo[1,2-a]pyridin. Es ist bekannt, daß Derivate solcher Imidazo[1,2-a]pyridine pharmakologische Wirkungen besitzen (J.Med.Chem. (1969), 122-26; Eur.J.Med.-Chim. Therapeut. 13, 271-76 (1978)).

Da die erfindungsgemäßen Verbindungen der Formel I aber auch Analoga der Acetessigsäureamide darstellen, können sie - beispielsweise nach nukleophilem Austausch des Halogenatoms - als Kupplungskomponenten zur Herstellung von Azofarbstoffen bzw. -pigmenten eingesetzt werden.
Der folgende Versuchsbericht zeigt die Synthese eines solchen Azopigments:

Versuchsbericht

6,9 g (50 mMol) 4-Nitranilin wurden in einer Mischung von 12 ml konz. Salzsäure und 12 ml Wasser gelöst und auf 0°C abgekühlt. Die Lösung wurde mit 14 ml (53.3 mMol) einer 20 gew.-%igen Natriumnitritlösung versetzt und 15 Minuten bei 0°C nachgerührt. Danach wurde das überschüssige Natriumnitrit mit Amidosulfonsäure zerstört. Die Diazoniumsalzlösung wurde bei 5°C in eine Dispersion von 11.6 g (50 mMol) Bromacetonsulfonamid in 50 ml Wasser getropft und dabei wurde der pH-Wert unter 6 gehalten. Nach 6 Stunden wurde das ausgefallene Produkt abgesaugt und getrocknet. Man erhielt 8 g eines gelben Farbstoffes der Formel

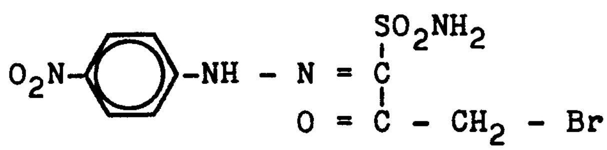

Der Vorteil dieses Azofarbstoffs liegt darin, daß man das aktivierte Halogenatom gegen Nukleophile austauschen und somit die Eigenschaften des Farbstoffs in gewünschter Weise verändern kann, und daß die saure Sulfonamidgruppe mit Salzen, z.B. des Bariums, Calciums oder Aluminiums verlackt werden kann, wodurch die Löslichkeit der Farbstoffe stark herabgesetzt wird, was für den Einsatz als Pigmente sehr erwünscht ist.

Die Fluor-, Chlor- bzw. Jodverbindung gemäß Formel I kann analog zur Bromverbindung zu einem Azopigment umgesetzt werden.

Die nachfolgenden Beispiele erläutern die Herstellung der erfindungsgemäßen Verbindungen:

Beispiel 1

137,2 g (1 Mol) Acetonsulfonamid wurden in 200 ml Wasser suspendiert und bei 20°C wurden unter Kühlung 160 g (1 Mol = 52 ml) Brom zugetropft (Zeitdauer ca. 30 Min.). Es wurde noch 1 Stunde bei Raumtemperatur nachgerührt. Das Lösungsmittel wurde am Rotationsverdampfer im Wasserstrahlvakuum abdestilliert. Der angefallene Rückstand wurde dann aus 1,5 l i-Propanol umkristallisiert. Man erhielt 186 g (86 % d.Th.) 3-Bromacetonsulfonamid vom Schmelzpunkt 119 - 120°C.

Elementaranalyse: $C_3H_6BrNO_3S$ (Molekulargewicht 216.05)

Berechnet: C: 16,7; H: 2,8; Br: 37,0; N: 6,5; S: 14,8

Gefunden: C: 17,0; H: 2,7; Br: 37,4; N: 6,5; S: 15,2

NMR-Spektrum: 100 MHz - $^1$HNMR (DMSO-$d_6$) (ppm):

4,4 (2H,s,$\underline{CH_2}$), 4,5(2H,s,$\underline{CH_2}$), 7,2(2H,s,$\underline{NH_2}$).

Beispiel 2

137,2g(1 Mol) Acetonsulfonamid wurden mit 200 ml Wasser auf 40°C erhitzt und bei dieser Temperatur innerhalb von 2 Stunden mit 1 Mol Brom versetzt. Es wurde noch 0,5 Stunden nachgerührt und wie in Beispiel 1 aufgearbeitet. Ausbeute: 181,6 g (84 % d.Th.), Schmelzpunkt: 117-119°C.

Beispiel 3

43,2 g (0,2 Mol) 3-Bromacetonsulfonamid wurden in 300 ml Aceton gelöst und mit 33,2 g (0,2 Mol) Kaliumjodid versetzt. Nach 5-stündigem Rühren bei Raumtemperatur wurde abgesaugt und mit Aceton nachgewaschen. Die Mutterlauge wurde im Wasserstrahlvakuum eingedampft und der Rückstand aus 500 ml i-Propanol umkristallisiert. Man erhielt 45 g (85 % d.Th.) 3-Jodacetonsulfonamid vom Schmelzpunkt 125-127°C.

Elementaranalyse: $C_3H_6JNO_3S$ (Molekulargewicht 263.05)

Berechnet: C: 13,7; H: 2,3; J: 48,2; N: 5,3; S: 12,2

Gefunden: C: 13,7; H: 2,2; J: 48,0; N: 5,6; S: 12,4

NMR-Spektrum: 100 MHz - $^1$HNMR (DMSO-$d_6$) (ppm): 4,2(2H,s,$\underline{CH_2}$), 4,4(2H,s,$\underline{CH_2}$), 7,2(2H,s,$\underline{NH_2}$).

Beispiel 4

4,3 g (20 mMol) 3-Bromacetonsulfonamid wurden in 30 ml Aceton gelöst und mit 3 g (40 mMol) Kaliumchlorid versetzt. Es wurde 1,5 Stunden bei Raumtemperatur gerührt, filtriert und mit Aceton gewaschen. Die Mutterlauge wurde am Rotationsverdampfer eingedampft. Der Rückstand, aus 30 ml 1-Propanol umkristallisiert, ergab 3 g Produkt vom Schmelzpunkt 110-112°C. Es wurde nochmals aus 30 ml Ethanol umkristallisiert, wobei man 2,0 g 3-Chloracetonsulfonamid vom Schmelzpunkt 114-115°C erhielt.
NMR-Spektrum: 100 MHz - $^1$HNMR (DMSO-$d_6$) (ppm): 4,3(2H,s,$\underline{CH_2}$), 4,7(2H,s,$\underline{CH_2}$), 7,2(2H,s,$\underline{NH_2}$).

Patentansprüche:

1. Verbindungen der Formel

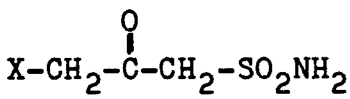

worin X = Fluor, Chlor, Brom oder Jod sein kann.

2. Verfahren zur Herstellung von 3-Bromacetonsulfonamid

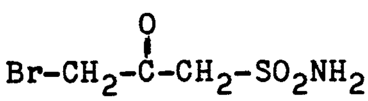

dadurch gekennzeichnet, daß man Acetonsulfonamid in wäßriger Lösung mit Brom umsetzt.

3. Verfahren zur Herstellung von Verbindungen der Formel

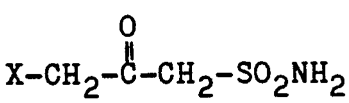

mit X = F, Cl oder J, dadurch gekennzeichnet, daß man 3-Brom-acetonsulfonamid in einem aprotischen Lösungsmittel mit einem Fluorid, Chlorid oder Jodid umsetzt.

4. Verwendung von Verbindungen gemäß Anspruch 1 zur Herstellung von Azofarbstoffen oder -pigmenten, Pflanzenschutzmitteln, Pharmazeutika und Textilhilfsmitteln.

Europäisches Patentamt

# EUROPÄISCHER RECHERCHENBERICHT

0218085

Nummer der Anmeldung

EP 86 11 1971

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 063 767 (HOECHST)<br>* Ansprüche *<br>--- | 1-4 | C 07 C 143/74 |
| A | CHEMICAL ABSTRACTS, Band 103, Nr. 21, 25. November 1985, Seite 680, Zusammenfassung no. 178236q, Columbus, Ohio, US; C. CARPANELLI et al.: "[4+2]-Cycloaddition reactions of N-sulfinylcarboxamides. III. Reactions of N-sulfinyl-p-toluamide with alkynes", & GAZZ. CHIM. ITAL. 1985, 115(5), 265-8<br>----- | 1 | |
| | | | RECHERCHIERTE SACHGEBIETE (Int. Cl.4) |
| | | | C 07 C 143/00 |

Der vorliegende Recherchenbericht wurde für alle Patentansprüche erstellt

| Recherchenort | Abschlußdatum der Recherche | Prüfer |
|---|---|---|
| DEN HAAG | 15-12-1986 | MOREAU J.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument